# FASCICULE DE BREVET EUROPEEN

(11) **EP 0 931 057 B1**
(45) Date de publication et mention de la délivrance du brevet: **21.11.2001**
(21) Numéro de dépôt: 97919122.8
(22) Date de dépôt: 17.09.1997
(51) Int. Cl.: C07C 253/30, C07D 257/04

(54) **PROCEDE POUR LA PREPARATION DE DERIVES DE 4-METHYL-BIPHENYLE**
VERFAHREN ZUR HERSTELLUNG VON 4-METHYL-BIPHENYL-DERIVATEN
METHOD FOR PREPARING 4-METHYL-BIPHENYL DERIVATIVES

(30) Priorité: 20.09.1996 FR 9611514
(43) Date de publication de la demande: 28.07.1999
(73) Titulaire: SANOFI-SYNTHELABO, 75635 Paris Cédex 13 (FR)
(72) Inventeur: ALAMI, Mouad, F-77000 Bussy Saint Georges (FR); CAHIEZ, Gérard, F-75003 Paris (FR); CASTRO, Bertrand, F-34130 Saint Aunes (FR); DORMOY, Jean-Robert, F-92330 Sceaux (FR); RIGUET, Eric, F-91920 Les Ulis (FR)
(74) Mandataire: Le Guen, Gérard
(86) Numéro de dépôt international: FR9701648
(87) Numéro de publication internationale: WO9812174

(56) Documents cités:
- EP-A- 0 540 356
- EP-A- 0 566 468
- WO-A-96/13489
- CHEMICAL ABSTRACTS, vol. 125, no. 7, 12 août 1996 Columbus, Ohio, US; abstract no. 86290, ASAI, TOMOYUKI ET AL: "Preparation of biphenyl compounds" XP002032661 & JP 08 109 143 A (ASAHI GLASS CO LTD, JAPAN)
- CHEMICAL ABSTRACTS, vol. 126, no. 1, 1 janvier 1997 Columbus, Ohio, US; abstract no. 7801, KOKUNI, NOBUKI ET AL: "Catalytic preparation of 4-methyl-2'-substituted-biphenyls by Grignard reaction" XP002032662 & JP 08 231 454 A (TOYO STAUFFER CHEM CO, JAPAN)
- RUSSELL, RONALD K. ET AL: "Efficient synthesis of 5-(4'-methyl[1,1'-biphenyl]-2-yl)-1H- tetrazole" J. ORG. CHEM. (1993), 58(18), 5023-4 CODEN: JOCEAH;ISSN: 0022-3263, 1993, XP000673994

## Description

La présente invention concerne un procédé de préparation de dérivés du biphényle. Plus particulièrement, l'invention a pour objet un procédé de préparation de dérivés de 4-méthyl-biphényle de formule générale : 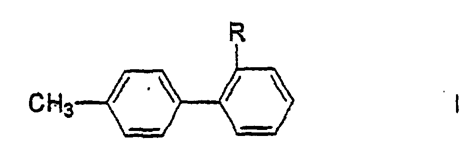 dans laquelle R représente un groupement cyano

Les dérivés de 4-méthyl-biphényle de formule I sont des composés connus particulièrement utiles comme intermédiaires dans la synthèse de nombreux principes actifs de médicaments agissant notamment contre l'hypertension par un mécanisme d'inhibition de l'angiotensine II.

Ainsi, les dérivés tétrazolyles de formule I ont été décrits dans WO 96/13489 tandis que le dérivé cyano de formule I c'est-à-dire l'o-(p-tolyl) benzonitrile, ci-après désigné plus brièvement orthotolylbenzonitrile ou OTBN a été décrit pour la première fois dans EP 253310.

Un certain nombre de procédés pour la synthèse de l'OTBN ont été récemment proposés. Le procédé qui semble être le plus approprié est décrit dans EP 566468 et consiste en la réaction entre un o-halobenzonitrile avec un halogénure de p-tolymagnésium en présence d'un sel manganeux, de préférence MnCl₂, cette réaction se déroulant en général dans un éther tel que le tétrahydrofurane, le dibutyl éther ou le dioxane.

Cette méthode, par rapport à celles précédemment connues, a l'avantage de se dérouler en une seule étape avec des rendements d'environ 70% avant cristallisation. Elle donne, cependant comme sous-produit de réaction, le 4,4-diméthylbiphényle résultant de la condensation de l'halogénure de p-tolylmagnésium sur lui-même.

D'autre part, on a rapporté dans EP 566468 en question des résultats d'essais d'orientation pour la préparation de l'OTBN à partir du bromure de p-tolylmagnésium et du 2-chloro-benzonitrile, la réaction étant effectuée dans le tétrahydrofurane en présence ou non de différents catalyseurs contenant un métal de transition à savoir PdCl₂, NiCl₂, Pd (PPh₃)₄. Ces essais se sont soldés par des rendements pauvres voire nuls en OTBN, selon les méthodes utilisées, puisque variant de 0 à 27%.

Or, il a maintenant été trouvé de manière surprenante que lorsque le couplage entre le o-halobenzonitrile et l'halogénure de p-tolylmagnésium est effectué en présence d'un polyéther, linéaire ou ramifié, et de traces d'un catalyseur contenant un métal de transition, l'OTBN est obtenu avec un rendement d'au moins environ 92% alors que l'impureté 4,4'-diméthylbiphényle chute en dessous d'environ 3,5%.

De tels résultats n'ont cependant pu être observés, lorsque le polyéther, linéaire ou ramifié, est remplacé en totalité par un diéther cyclique dont les deux oxygènes endocycliques font partie du même cycle, en l'occurrence le dioxane.

Ainsi, la présente invention a pour objet un procédé pour la préparation des composés de formule I en général et de l'o-(p-tolyl) benzonitrile en particulier, caractérisé en ce que l'on fait réagir un halobenzène de formule: 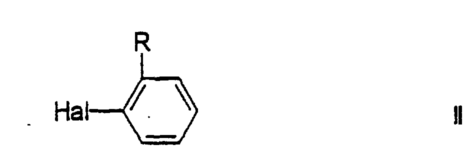 dans laquelle Hal représente un atome d'halogène, de préférence brome et R a la même signification que précédemment, avec un halogénure de p-tolylmagnésium en présence d'un polyéther, linéaire ou ramifié, et d'un catalyseur contenant un métal de transition.

Par polyéther, linéaire ou ramifié, on entend désigner tout composé organique comprenant au moins deux fonctions éther formant partie d'un cycle ou d'une chaîne hydrocarbonée, linéaire ou ramifiée, à l'exclusion de composés dont toutes les fonctions éther sont endocycliques et font partie d'un même cycle.

Selon un mode de réalisation préféré, le polyéther, linéaire ou ramifié, est un diéther linéaire ou ramifié, dont les deux fonctions éther, lorsqu'elles sont toutes deux endocycliques, ne font pas partie du même cycle.

De manière avantageuse, le diéther linéaire ou ramifié est tel que ses deux fonctions éther sont incorporées dans une chaîne hydrocarbonée, linéaire ou ramifiée, de préférence en C₂-C₁₂, mieux encore en C₂-C₆.

La réaction de couplage, selon l'invention, est effectuée dans un milieu constitué d'un polyéther, linéaire ou ramifié, auquel on a éventuellement ajouté un solvant du type monoéther tel que le méthyl-t-butyl éther ou le dibutyl éther ou encore un mono-ou di-éther cyclique tel que le dioxane ou le tétrahydrofurane, la température de réaction pouvant varier de -10 à 65°C, selon le milieu employé.

On a, en fait, trouvé, que pour améliorer le déroulement de la réaction en question il est essentiel de procéder en présence d'un polyéther de ce type généralement un diéther glycolique. Selon l'invention, un éther glycolique est un éther de glycol dans lequel le glycol est constitué d'une chaîne hydrocarbonée dihydroxylée, linéaire ou ramifiée de préférence en C₂-C₁₂, mieux encore en C₂-C₆. Les éthers de 1,2-glycol et notamment le diéthylène glycol sont plus particulièrement avantageux.

A ce titre le diéthoxyéthane et, de préférence le diméthoxyéthane, se sont révélés particulièrement intéressants.

Cette réaction de couplage conduit transitoirement à la formation d'un complexe que l'on hydrolyse selon les procédures habituelles, par exemple au moyen d'un acide tel que l'acide chlorhydrique.

Le métal de transition formant le catalyseur est avantageusement le cobalt, le nickel, le platine, le manganèse ou notamment le palladium.

Comme catalyseur contenant un métal de transition, on utilise préférentiellement un sel de palladium(II), notamment le nitrate, le chlorure, l'acétate, le bromure, le sulfate ou analogues, le chlorure (PdCl₂) et l'acétate (CH₃-COO-Pd-OOC-CH₃) étant particulièrement avantageux. De préférence, le sel de palladium est complexé par exemple avec au moins un composé organophosphoré comprenant du phosphore. trivalent. Plus particulièrement, on utilise des complexes de palladium, tels que les bis(triphénylphosphine) dichloro-, bis(tributylphosphine)dichloro-, bis(tricyclohexylphosphine)dichloro-, diallyltriphénylphosphinedichloro-, triphénylphosphinepipéridino dichloro-, bis(cyclohexyloxime)dicarbonyl-, 1,5,9-cyclododécatriènedichloro-, bis-triphénylphosphine)dicarbonyl-, bis(triphénylphosphine)diacétate-, bis(triphénylphosphine)sulfate-, 2,4-pentanedione, tétrakis(triphénylphosphine)-palladium. Parmi ceux-ci, les complexes de palladium (II) sont particulièrement avantageux, le 1,3-bis(diphénylphosphino) propane (dppp) complexé avec le chlorure de palladium (II) ou l'acétate de palladium (II) étant préféré.

Les sels de palladium et les composés organophosphorés peuvent être ajoutés séparément au milieu réactionnel. Dans ce cas, la quantité de composé organophosphoré est de préférence suffisante pour former *in situ* le catalyseur sous forme de complexe avec le palladium présent.

Ledit complexe est en général préparé de façon à ce que le rapport P/Pd soit environ 1/1, mais un tel rapport peut varier entre 0,5/1 et 2/1 sans altérer significativement le résultat du procédé.

Ce catalyseur est présent en très faibles quantités dans le mélange réactionnel, à savoir de 0,001 à 2% molaire par mole de o-halobenzonitrile de départ.

Selon un mode opératoire préférentiel, l'halogénure de p-tolymagnésium est en quantités équimolaires ou en faible excès (1 à 1,7 mole) par rapport à l'ohalobenzonitrile.

En outre, la réaction peut être conduite dans le tétrahydrofurane contenant le diméthoxyéthane en ajoutant, à la température de 10°C, le catalyseur et l'o-halobenzonitrile éventuellement en solution dans le tétrahydrofurane à une solution de tétrahydrofurane contenat l'halogénure de p-tolylmagnésium. Cette réaction qui est exothermique, peut être contrôlée en réglant la vitesse d'addition du dérivé de benzonitrile et du catalyseur de façon à la maintenir inférieure à 35°C.

D'une manière alternative, la réaction peut être également conduite en ajoutant l'halogénure de p-tolylmagnésium dans, par exemple, le tétrahydrofurane à un mélange d'o-halobenzonitrile et de catalyseur dans, par exemple, le tétrahydrofurane contenant le diméthoxyéthane. Dans ce cas, la température de réaction peut être mieux contrôlée et l'addition d'halogénure de p-tolylmagnésium peut être effectuée même à une température plus élevée, de l'ordre de 60-65°C, de façon à diminuer la durée de la réaction et la quantité de catalyseur employée.

Selon le mode opératoire préférentiel ci-dessus, l'hydrolyse est effectuée *in situ* par de l'acide chlorhydrique et l'OTBN ainsi formé est isolé selon les techniques conventionnelles, par exemple par extraction avec un solvant convenable, évaporation du solvant et purification par cristallisation dans de l'éthanol ou par chromatographie.

L'OTBN est ainsi obtenu avec des rendements très élevés, de 92 à 98% selon les proportions des réactifs employés. Il contient des quantités très faibles de 4,4'-diméthylbiphényle, généralement inférieures à 3,5%.

La quantité de 4,4'-diméthylbiphényle qui se forme selon le procédé de la présente invention a été comparée à celle qui se forme selon le procédé décrit dans EP 566468. Ainsi en opérant :
- selon EP 566468, à savoir en utilisant le seul MnCl2 en tant que catalyseur, dans une série d'essais dans les mêmes conditions, on a obtenu du sous-produit 4,4'-diméthylbiphényle avec un rendement de 8 à 12% par rapport au bromure de tolylmagnésium, soit 6,5 à 10% en poids de 2-(p-tolyl)benzonitrile produit final ;
- selon la présente invention, à savoir en présence de diméthoxyéthane et en utilisant PdCl₂/dppp en tant que catalyseur, dans une série d'essais dans les mêmes conditions, on a obtenu du sous-produit 4,4'-diméthylbiphényle avec un rendement de 0,5 à 1% par rapport au bromure de p-tolylmagnésium, soit au maximum 0,65% en poids de produit final.

Le catalyseur contenant un métal de transition peut être également un sel de cobalt, de nickel, de platine ou de manganèse comme indiqué précédemment.

Dans le cas d'un catalyseur contenant du nickel, on utilise généralement un sel de nickel (II) tel que le chlorure ou l'acétylacétonate de nickel. Ce sel est préférentiellement complexé avec au moins un composé organophosphoré comprenant du phosphore trivalent tel qu'une phosphine, par exemple la triphénylphosphine. Le sel de nickel et le composé organophosphoré peuvent être ajoutés séparément au milieu réactionnel.

Ce catalyseur contenant du nickel est avantageusement prétraité avec un agent réducteur tel qu'un hydrure par exemple l'hydrure de dibutylaluminium ou l'hydrure de diisobutylaluminium ou encore avec un halogénure de méthylmagnésium par exemple le chlorure de méthylmagnésium, de manière à former des catalyseurs contenant du Ni(O) tel que Ni[P(C₆H₅)₃]₄.

Des systèmes comprenant l'acétylacétonate de nickel, la triphénylphosphine et l'hydrure de diisobutylaluminium se sont révélés particulièrement intéressants.

Quant au sel de manganèse, généralement un sel manganeux, il s'agit de préférence de MnCl₂ ou de MnCl₄Li₂, ce dernier pouvant être formé *in situ* par addition de deux équivalents molaires de LiCI et d'un équivalent molaire de MnCl₂.

Ces catalyseurs formés par des sels de cobalt, de nickel, de platine ou de manganèse peuvent être mis en oeuvre, dans le procédé de l'invention de manière semblable à celle décrite ci-dessus pour les sels de palladium (II).

Comme indiqué précédemment, les dérivés de 4-méthyl-biphényle de formule I peuvent être utilisés pour la préparation de médicaments antagonistes de l'angiotensine II.

Des méthodes pour la préparation de ces médicaments au départ des composés de formule I ont été largement décrites. A ce titre, on citera par exemple WO 96/13489, EP 253310, EP 324377 ou EP 454511.

L'Exemple non limitatif suivant illustre l'invention. Dans cet exemple, les pourcentages molaires en catalyseur sont calculés par rapport à la quantité d'ortho-halobenzonitrile.

### EXEMPLE

### Préparation du o-(p-tolyl)benzonitrile

Sous atmosphère d'azote, on ajoute successivement, à 7 ml de tétrahydrofurane anhydre, 4 équivalents molaires de diméthoxyéthane (environ 2 ml), du PdCl₂/dpp : 1/1 (0,023 g, 1 mol %) puis l'o-bromobenzonitrile (0,72 g, 3,955 mol). On agite le mélange pendant 5 minutes puis on le chauffe à 65°C. On ajoute alors en 7 minutes une solution de chlorure de p-tolylmagnésium dans le tétrahydrofurane (1N; 6,73 ml; 6,73 mmol).

Après 5 minutes d'agitation à 65°C, on refroidit le milieu réactionnel à la température ambiante puis on l'hydrolyse à l'aide d'une solution d'acide chlorhydrique 1N (15 ml). Après extraction à l'éther éthylique, on sèche la phase organique sur carbonate de potassium, on filtre puis on évapore sous vide. L'huile obtenue est purifiée par chromatographie (silice : 20 g; éluant : éther de pétrole / acétate d'éthyle : 95/5). Le o-(p-tolyl)benzonitrile est ainsi obtenu avec un rendement de 93%, sous forme de cristaux blanchâtres.

## Revendications

1. Procédé pour la préparation de dérivés de 4-méthyl-biphényle de formule générale : 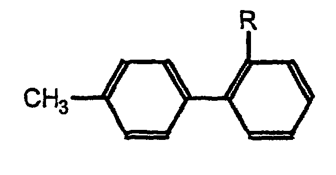 dans laquelle R représente un groupement cyano, ledit procédé assurant une réduction de la formation de l'impureté 4,4'-diméthylbiphényle, **caractérisé en ce que** l'on fait réagir un halobenzène de formule : 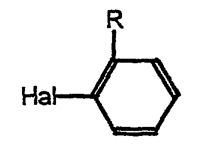 dans laquelle Hal représente un atome d'halogène et R a la même signification que précédemment, avec un halogénure de p-tolymagnésium en présence d'un polyéther, linéaire ou ramifié, et d'un catalyseur contenant un métal de transition, étant entendu que ledit polyéther linéaire ou ramifié désigne tout composé organique comprenant au moins deux fonctions éther formant partie d'un cycle ou d'une chaîne hydrocarbonée, linéaire ou ramifiée, à l'exclusion de composés dont toutes les fonctions éther sont endocycliques et font partie d'un même cycle.

2. Procédé selon la Revendication 1 **caractérisé en ce que** Hal représente le brome.

3. Procédé selon une des Revendications 1 à 2, **caractérisé en ce que** le catalyseur contenant un métal de transition est un sel de palladium, un sel de cobalt, un sel de nickel, un sel de platine ou un sel de manganèse.

4. Procédé selon une des Revendications 1 à 3 **caractérisé en ce que** le catalyseur contenant un métal de transition est un sel de palladium (II).

5. Procédé selon la Revendication 4, **caractérisé en ce que** le sel de palladium (II) est le chlorure de palladium (II) ou l'acétate de palladium (II).

6. Procédé selon une des Revendications 3 à 5, **caractérisé en ce que** le sel de palladium est ajouté au milieu réactionnel avec un composé organophosphoré comprenant du phosphore trivalent.

7. Procédé selon une des Revendications 3 à 6 **caractérisé en ce que** le sel de palladium est sous forme de complexe avec un composé organophosphoré comprenant du phosphore trivalent.

8. Procédé selon la Revendication 7, **caractérisé en ce que** le sel de palladium est sous forme de complexe avec le 1,3-bis(diphénylphosphino)propane et le chlorure ou l'acétate de palladium (Il).

9. Procédé selon la Revendication 3, **caractérisé en ce que** le sel de nickel est le chlorure de nickel ou l'acétylacétonate de nickel.

10. Procédé selon la Revendication 3 ou 9, **caractérisé en ce que** le sel de nickel est ajouté au milieu réactionnel avec un composé organophosphoré comprenant du phosphore trivalent.

11. Procédé selon une des Revendications 3, 9 ou 10, **caractérisé en ce que** le sel de nickel est sous forme de complexe avec un composé organophosphoré comprenant du phosphore trivalent.

12. Procédé selon une des Revendications 3, 9, 10 où 11, **caractérisé en ce que** le sel de nickel est prétraité avec un agent réducteur.

13. Procédé selon une des Revendications 1 à 12, **caractérisé en ce que** le polyéther, linéaire ou ramifié, est un diéther glycolique.

14. Procédé selon une des Revendications 1 à 13 **caractérisé en ce que** le polyéther, linéaire ou ramifié, est le diméthoxyéthane.

15. Procédé selon une des Revendications 1 à 14, **caractérisé en ce que** le milieu réactionnel contient un solvant du type monoéther ou mono- ou di- éther cyclique.

16. Procédé selon la Revendication 15 **caractérisé en ce que** le solvant du type monoéther est le méthyl-t-butyl éther ou le dibutyl éther, et le solvant du type mono- ou di- éther cyclique est le tétrahydrofurane ou le dioxane.

## Patentansprüche

1. Verfahren zur Herstellung von 4-Methyl-biphenyl-Derivaten der allgemeinen Formel: 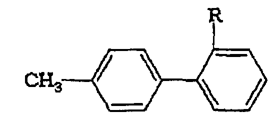 in der R eine Cyanogruppe bedeutet, welches Verfahren die Verminderung der Bildung von 4,4'-Dimethylbiphenyl-Verunreinigungen sicherstellt, **dadurch gekennzeichnet, daß** man ein Halogenbenzol der Formel: 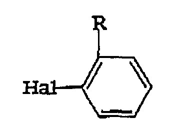 in der Hal ein Halogenatom und R die oben angegebenen Bedeutungen besitzt, mit einem p-Tolylmagnesiumhalogenid in Gegenwart eines geradkettigen oder verzweigten Polyethers und eines Katalysators, der ein Übergangsmetall enthält, umsetzt, wobei der geradkettige oder verzweigte Polyether für jede organische Verbindung steht, die mindestens zwei Etherfunktionen aufweist, die Teil eines Ringes oder einer geradkettigen oder verzweigten Kohlenwasserstoffkette sind, mit Ausnahme von Verbindungen, bei denen sämtliche Etherfunktionen endocyclisch und Teil eines gleichen Ringes sind.

2. Verfahren nach Anspruch 1, **dadurch gekennzeichnet, daß** Hal für Brom steht.

3. Verfahren nach einem der Ansprüche 1 bis 2, **dadurch gekennzeichnet, daß** der ein Übergangsmetall enthaltende Katalysator ein Palladiumsalz, ein Kobaltsalz, ein Nickelsalz, ein Platinsalz oder ein Mangansalz ist.

4. Verfahren nach einem der Ansprüche 1 bis 3, **dadurch gekennzeichnet, daß** der ein Übergangsmetall enthaltende Katalysator ein Palladium(II)-salz ist.

5. Verfahren nach Anspruch 4, **dadurch gekennzeichnet, daß** das Palladium(II)-salz Palladium(II)-chlorid oder Palladium(II)-acetat ist.

6. Verfahren nach einem der Ansprüche 3 bis 5, **dadurch gekennzeichnet, daß** das Palladiumsalz zusammen mit einer phosphororganischen Verbindung, die dreiwertigen Phosphor enthält, zu dem Reaktionsmedium zugegeben wird.

7. Verfahren nach einem der Ansprüche 3 bis 6, **dadurch gekennzeichnet, daß** das Palladiumsalz in Form eines Komplexes mit einer phosphororganischen Verbindung, die dreiwertigen Phosphor enthält, vorliegt.

8. Verfahren nach Anspruch 7, **dadurch gekennzeichnet, daß** das Palladiumsalz in Form eines Komplexes mit 1,3-Bis(diphenylphosphino)-propan und Palladium(n)-chlorid oder -acetat vorliegt.

9. Verfahren nach Anspruch 3, **dadurch gekennzeichnet, daß** das Nickelsalz Nickelchlorid oder Nickelacetylacetonat ist.

10. Verfahren nach Anspruch 3 oder 9, **dadurch gekennzeichnet, daß** das Nickelsalz zusammen mit einer phosphororganischen Verbindung, die dreiwertigen Phosphor enthält, zu dem Reaktionsmedium zugesetzt wird.

11. Verfahren nach einem der Ansprüche 3, 9 oder 10, **dadurch gekennzeichnet, daß** das Nickelsalz in Form eines Komplexes mit einer phosphororganischen Verbindung, die dreiwertigen Phosphor enthält, vorliegt.

12. Verfahren nach einem der Ansprüche 3, 9, 10 oder 11, **dadurch gekennzeichnet, daß** das Nickelsalz mit einem Reduktionsmittel vorbehandelt worden ist.

13. Verfahren nach einem der Ansprüche 1 bis 12, **dadurch gekennzeichnet, daß** der geradkettige oder verzweigte Polyether ein Glykoldiether ist.

14. Verfahren nach einem der Ansprüche 1 bis 13, **dadurch gekennzeichnet, daß** der geradkettige oder verzweigte Polyether Dimethoxyethan ist.

15. Verfahren nach einem der Ansprüche 1 bis 14, **dadurch gekennzeichnet, daß** das Reaktionsmedium ein Lösungsmittel vom Typ eines Monoethers oder eines cyclischen Mono- oder Diethers enthält.

16. Verfahren nach Anspruch 15, **dadurch gekennzeichnet, daß** das Lösungsmittel vom Typ des Monoethers Methyl-tert.-butylether oder Dibutylether ist und das Lösungsmittel vom Typ des cyclischen Mono- oder Diethers Tetrahydrofuran oder Dioxan ist.

## Claims

1. Process for preparing 4-methyl-biphenyl derivatives of general formula: 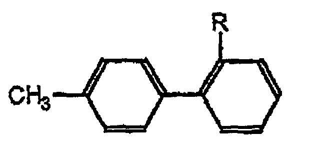 wherein R denotes a cyano group, said process ensuring a reduction in the formation of the impurity 4,4'-dimethylbiphenyl, **characterised in that** a halobenzene of formula: 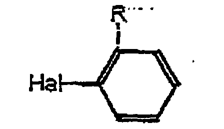 wherein Hal denotes a halogen atom and R has the same meaning as hereinbefore, is reacted with a p-tolyl magnesium halide in the presence of a straight-chain or branched polyether and a catalyst containing a transition metal, with the proviso that said straight-chain or branched polyether denotes any organic compound having at least two ether functions forming part of a ring or a straight-chain or branched hydrocarbon chain, with the exception of compounds in which all the ether functions are endocyclic and are part of the same ring.

2. Process according to claim 1, **characterised in that** Hal denotes bromine.

3. Process according to one of claims 1 and 2, **characterised in that** the catalyst containing a transition metal is a palladium salt, a cobalt salt, a nickel salt, a platinum salt or a manganese salt.

4. Process according to one of claims 1 to 3, **characterised in that** the catalyst containing a transition metal is a palladium (II) salt.

5. Process according to claim 4, **characterised in that** the palladium (II) salt is palladium (II) chloride or palladium (II) acetate.

6. Process according to one of claims 3 to 5, **characterised in that** the palladium salt is added to the reaction medium with an organophosphorus compound containing trivalent phosphorus.

7. Process according to one of claims 3 to 6, **characterised in that** the palladium salt is in the form of a complex with an organophosphorus compound containing trivalent phosphorus.

8. Process according to claim 7, **characterised in that** the palladium salt is in the form of a complex with 1,3-bis(diphenylphosphino)propane and palladium (II) chloride or acetate.

9. Process according to claim 3, **characterised in that** the nickel salt is nickel chloride or nickel acetyl acetonate.

10. Process according to claim 3 or 9, **characterised in that** the nickel salt is added to the reaction medium with an organophosphorus compound containing trivalent phosphorus.

11. Process according to claim 3, 9 or 10, **characterised in that** the nickel salt is in the form of a complex with an organophosphorus compound containing trivalent phosphorus.

12. Process according to claim 3, 9, 10 or 11, **characterised in that** the nickel salt is pretreated with a reducing agent.

13. Process according to one of claims 1 to 12, **characterised in that** the straight-chain or branched polyether is a glycol diether.

14. Process according to one of claims 1 to 13, **characterised in that** the straight-chain or branched polyether is dimethoxyethane.

15. Process according to one of claims 1 to 14, **characterised in that** the reaction medium contains a solvent of the monoether or cyclic mono- or diether type.

16. Process according to claim 15, **characterised in that** the solvent of the monoether type is methyl-t-butyl ether or dibutyl ether, and the solvent of the cyclic mono- or diether type is tetrahydrofuran or dioxane.
